# EUROPEAN PATENT APPLICATION

(11) **EP 3 167 904 A1**
(43) Date of publication of application: **17.05.2017**
(21) Application number: 16198320.0
(22) Date of filing: 11.11.2016
(51) Int. Cl.: A61L 2/07, E06B 3/46, E05D 15/06

(54) **AUTOCLAVE DOOR AND METHOD FOR ITS MANUFACTURING**

(30) Priority: 13.11.2015 IT UB20155580
(71) Applicant: Angelantoni Life Science S.r.l., 06056 Massa Martana (PG) (IT)
(72) Inventor: VALENTINI, Giovanni, I-06056 Massa Martana PG (IT); DE PIAN, Eros, I-06056 Massa Martana PG (IT)
(74) Representative: Ercolani, Simone Pietro

(57) **Abstract**

Device (1) for opening/closing an autoclave (100) having an opening (101) for inputting/outputting one or more products to be sterilized, said device (1) comprising a frame (2) arranged at the edges (101a, 101b, 101c, 101d) of said opening (101), and at least one door (3) combinable with said frame (2) by sliding between a closed position and an open position, characterized in that said frame (2) is of framing type and comprises at least one first part (2a) arranged on a first edge (101a) of said opening (101) and provided with at least one linear guide (10) operatively facing the outer side of said opening, and in that said door (3) comprises a central body (20) and at least one first portion (21) provided with at least one first linear profile (22) operatively facing the inside of said opening and sliding along said guide of said at least one first part, said at least one first portion being distinct from said central body and being constrained along at least one first end (20a) of said central body (20).

## Description

### FIELD OF THE INVENTION

The present invention concerns a device for opening/closing autoclaves and respective manufacturing method.

In particular, the present invention is suitable to be used especially in autoclaves used for sterilizing products and in which the opening/closing device is of sliding type.

### KNOWN PREVIOUS ART

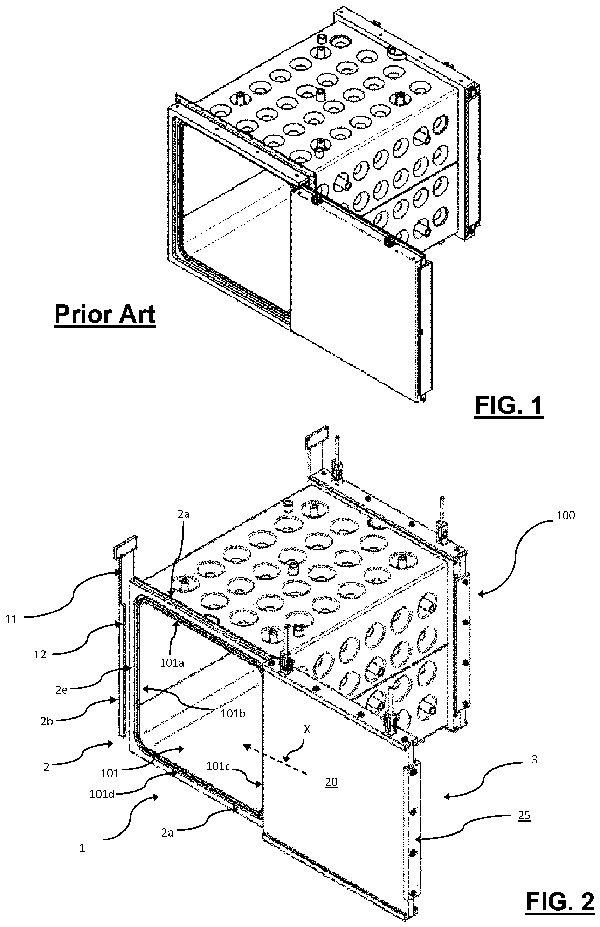

In fact, according to known art, an autoclave is provided with an opening usually lying on a plane and suitable to allow one or more products to be sterilized to be input/output to/from the autoclave, and with an opening/closing device. This opening/closing device comprises a frame constrained at the edges of the opening, and a door combinable with the frame by sliding along a direction parallel to the plane on which the opening of the autoclave lies. It is therefore clear that, when is in its closing position, the door must guarantee the perfect seal by cooperating with the frame, in order to prevent the steam in the autoclave, when the latter is operating, from leaking out thus endangering and damaging those who work near the autoclave. Still according to the art and as better shown in fig. 1, in order to guarantee this seal, the frame is provided with two first parts opposite and parallel to one another and comprising on their inner side, i.e. towards the opening, respective U-shaped linear guides in which two profiles slide, which are directly obtained on the profile of the door through standard machining. Furthermore, at a third part of the frame arranged orthogonally to the above mentioned two opposite parts, there is a third U-shaped groove also arranged on the inner side of the frame, i.e. towards the opening, and couplable to a third profile on the door. This U-shaped groove, which is internally joined to both the U-shaped linear guides that are on the two first opposite parts, is useful for keeping the sealing when the door is closed, as well as for acting as ending element of abutment for the door itself, and for limiting the door displacement in a direction orthogonal to the plane on which the opening lies. For this purpose, among other things, a hook-shaped element running along the whole edge of the door and shaped so as to couple to a further U-shaped groove obtained along a fourth part of the frame opposite to the above mentioned third part, is arranged on the door on the opposite side with respect to the abutment side.

However, such opening/closing device of known art is not free from drawbacks. In fact, although it is operatively able to keep a perfect seal between the door and the frame when the door is closed, and is able to minimize any displacement of the door in a direction orthogonal to the plane on which the opening lies, however special and high-precision workings are required for producing both the U-shaped guides and the groove that are arranged on the inner side of the frame. Due to this drawback, a considerable expenditure of energy is required in the production process, with long times and high costs.

As a result of the tolerances to be provided for the linear guides and the grooves of the frame, particularly accurate and therefore expensive technological workings are required also for the door itself.

Furthermore, as a result of the continuous opening and closing operations, the opening/closing devices of the known art are also subject to an appreciable level of wear over time, caused by not perfect balance of the door with respect to the frame. It is therefore an object of the present invention to provide a device for closing/opening autoclaves which is easier to be manufactured and assembled.

It is further object of the present invention to provide a device for opening/closing autoclaves that is more balanced and, therefore, over time and as a result of continuous door opening and closing operations, is subject to a lower level of wear.

### SUMMARY OF THE INVENTION

These and other objects are achieved by a device for opening/closing an autoclave having an opening for inputting/outputting one or more products to be sterilized, said device comprising a frame arranged at the edges of said opening, and at least one door combinable with said frame by sliding between a closed position and an open position, advantageously said frame is of the framing type and comprises at least one first part arranged on a first edge of said opening and provided with at least one linear guide operatively facing the outer side of said opening. Moreover, said door in turn comprises a central body and at least one first portion provided with at least one first linear profile operatively facing the inside of said opening and sliding along said linear guide of said at least one first part, in which said at least one first portion is distinct from said central body and is constrained along at least one first end of said central body. As evident, such a solution allows the proposed purposes to be achieved. In fact, thanks to the fact that the first part of the frame is provided with at least one linear guide operatively facing the outer side of said opening and that the door comprises a central body and at least one first portion provided with at least one first linear profile operatively facing the inside of said opening and sliding along said linear guide of said at least one first part, as well as the fact that said at least one first portion is distinct from said central body and is constrained along at least one first end of said central body, it is possible to obtain a device for opening/closing autoclaves not only able to perform usual tasks, such as sealing and mechanical strength, but also requiring much easier and quicker workings with respect to what provided for devices of known art. In this way, a considerable reduction of working time and costs is achieved. Additionally, the door is also more balanced than those of known art, thus minimizing the wear of the same due to normal opening-and-closing cycles of the door.

Furthermore, said frame comprises at least one second part arranged at a second edge of said opening and provided with at least one abutment surface to limit the sliding of said door in the closed position thereof. Moreover, said at least one abutment surface is constrained to said at least one second part of said frame. With this solution it is possible to obtain a frame from components whose implementation, as well as the respective technological workings are made simple.

According to an embodiment of the invention, said central body is further provided with at least one second end, wherein said at least one second end is shaped to be combined with said abutment surface of said at least one second part, at least when said door is in its closed position.

In a particular manner, said central body comprises a simple plate with constant thickness.

Still according to an embodiment of the invention, said at least one abutment surface comprises at least one hook element jutting from said abutment surface and adapted to limit the displacement of said door along a direction orthogonal to the sliding one, at least when said door is in its closed position. In particular, said at least one hook element is arranged from the outer planar surface of said frame at a distance substantially equal to the thickness of said central body.

Finally, said frame comprises a third part arranged on a third edge of said opening and provided with a groove operatively facing the outer side of said opening; at the same time, said door comprises at least one additional portion constrained on a third end of said central body and provided with another hook element operatively facing the inside of said opening and couplable by abutment with said groove of said at least one third part in order to limit the displacement of said door along a direction orthogonal to the sliding one, when said door is in its closed position. Advantageously, said at least one guide of said frame is of U-shaped type and said at least one linear profile of said at least one first portion of said door is shaped to slide in said U-shaped profile.

In a particular way, said at least one first portion and said at least one additional portion comprise a U-section having the same size and cross shape.

Still according to the invention, the objects are achieved also by a method for making an opening/closing device according to one or more of claims 1 to 9, comprising the step of a) making said frame of framing type and the step of b) making said door slidingly combinable with said frame, characterized in that said step a) comprises the step of a1) making at least one linear guide operatively facing the outer side of said opening at said at least one first part of said frame; and in that the step b) comprises the steps of: b1) making a plate with substantially constant thickness to form said central body; the step of b2) making at least one first portion provided with at least one first linear profile operatively facing the inside of said opening and sliding along said guide of said at least one first part, at least when the door is assembled; and the step of b3) constraining said at least one first portion on at least one first end of said central body.

Still according to the invention before said step a1), the step a) further comprises the step of a0) making a substantially quadrangular and central hole on a plate with substantially constant thickness.

Additionally and alternatively, before said step a1), the step a) further comprises the step of a0') welding two first linear parts with a second part and a third part, one to another, to form said frame of framing type.

Also, after said step a1) there is the step of a2) combining at least one abutment surface with said second part of said frame, in order to limit the sliding of said door in the closed position thereof.

Still according to the method, said step a) comprises the step of a3) making at least one groove operatively facing the outer side of said opening at said at least one third part, and in that said step b) further comprises the step of b4) making at least one additional portion provided with at least one additional hook element operatively facing the inside of said opening and couplable by abutment with said groove of said at least one third part, in order to limit the displacement of said door along a direction orthogonal to the sliding one, when said door is in its closed position, and the step of b5) constraining said at least one additional portion to a third end of said central body.

According to a preferred embodiment of the invention, said at least one first portion and said at least one additional portion are made from a conveniently cut U-section having the same size and cross shape.

In this way, the manufacture of the door is made much easier because both the working for making the plate-shaped central body with constant thickness and the working to obtain the linear profiles, are not particularly complex.

Furthermore, the two first portions and the additional portion comprise a U-section having the same size and cross shape. In this way, thanks to the particular U-shape, the two first portions as well as the additional portion may be constrained to the respective ends of the central body without requiring any special cautions, but using one of the two legs of the U-section as centering element and by mounting it by the concave part.

In a peculiar manner, it should be clarified that said at least one first linear guide is made along the whole extent of said at least one first part.

### BRIEF DESCRIPTION OF THE FIGURES

These and other aspects of the present invention will be made more clear by the following detailed description of a preferred embodiment, herein provided by way of example only and without limitations, with reference to the accompanying figures, in which:
- figure 1 shows an autoclave of known art;
- figure 2 shows an axonometric view of an autoclave provided with an opening/closing device according to the invention, with the door in its open position;
- figure 3 shows a further axonometric view of the autoclave of figure 2 with the door in its closed position;
- figure 4 shows an axonometric view of the door depicted in figures 2 and 3;

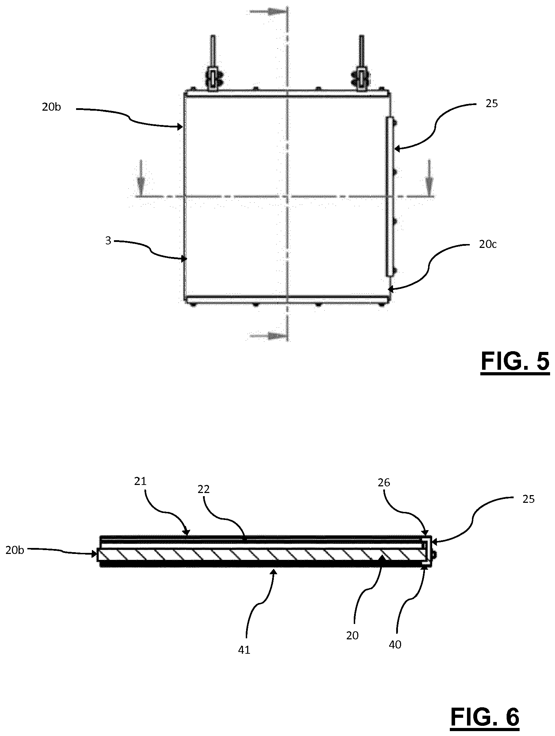

- figure 5 shows a front view of the door depicted in figure 4;
- figure 6 shows a sectional view of the door depicted in figure 4;

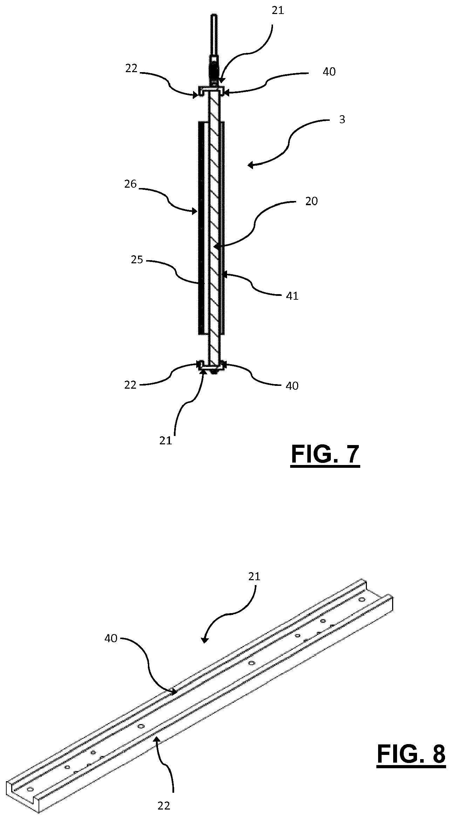

- figure 7 shows a further sectional view of the door depicted in figure 4;
- figure 8 shows an axonometric view of the first portion constrainable to the central body of the door;
- figure 9 shows an axonometric view of the frame of framing type only, without the abutment surface;
- figure 10a shows a front view of the frame of figure 9;
- figure 10b shows a sectional view of the frame of figure 10a.

### DETAILED DESCRIPTION OF A PREFERRED EMBODIMENT OF THE PRESENT INVENTION

Referring to the above figures, a device 1 for opening/closing an autoclave 100 is depicted, the device comprising an opening 101 for inputting/outputting one or more products to be sterilized such as, for example, gauzes, general surgical instruments and other sterilizable products for hospital use. However, it should be mentioned that the autoclave in question could also be used to sterilize other products, even not for hospital use, without departing from the protection scope of the present invention.

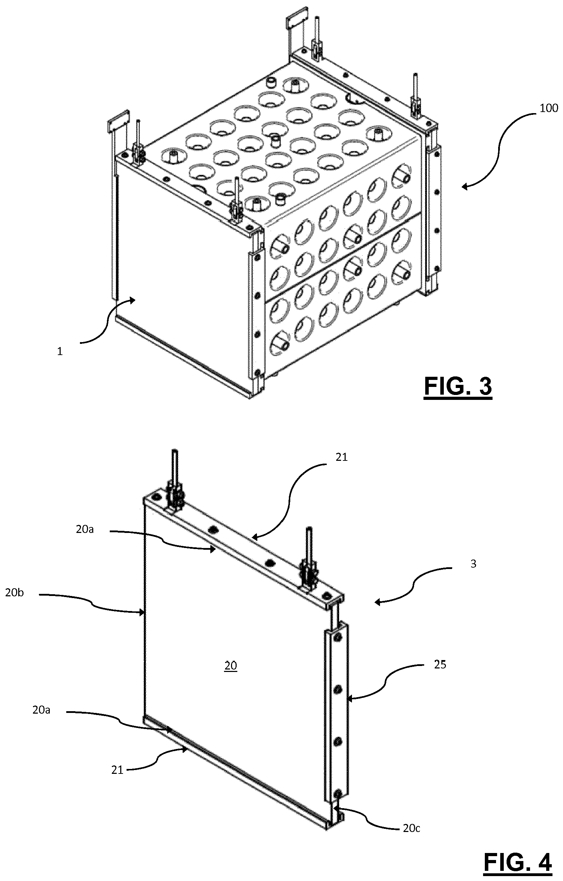

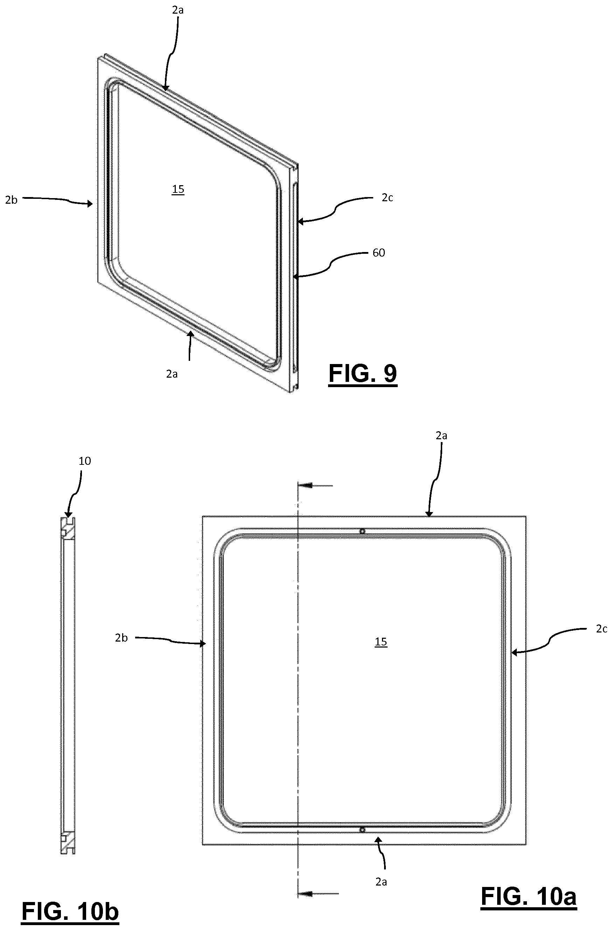

In particular, the device 1 comprises a frame 2 arranged at the edges 101a, 101b, 101c, 101d of the opening 101, and a door 3 combinable with the frame 2 by sliding between a closed position (figure 3) and an open position (figure 2), and vice versa. According to the invention, the frame 2 is of framing type and comprises two first parts 2a opposite and parallel to one another, which are arranged on a respective edge 101a and 101d of the opening 101. Each first part 2a is provided with a U-shaped linear guide 10 operatively facing the outer side of the opening 101 (see figures 9, 10a and 10b). Furthermore, the door 3 comprises a central body 20 comprising in turn a plate with constant thickness, and two first portions 21 each provided with a first linear profile 22 operatively facing the inside of the opening 101 and sliding along the respective U-shaped linear guide 10 of the first two parts 2a. Advantageously, the two first portions 21 are distinct from the central body 20 and are constrained by screws along the respective first ends 20a opposite and parallel to each other, of the central body 20. In practice, thanks to such a solution, not only it is possible to make a more simple frame 2, i.e. without working it internally but only on the outer side with respect to the opening, but the door 3 is made in several parts, each requiring to be very simply worked and not requiring particularly strict tolerances.

Still according to the embodiment described herein, the frame 2 comprises a second part 2b arranged at a second edge 101b of the opening, orthogonal to the edges 101a and 101b, and provided with an abutment surface 11 in order to limit the sliding of the door 3 in the closed position thereof. In particular, this abutment surface 11 is arranged, i.e. constrained on this second part 2b of the frame 2. Even in this case, therefore, the working made on the frame 2 is extremely simple. Moreover, also in this case the frame 2 is obtained by joining together components whose manufacturing is extremely simplified.

Additionally, the central body 20 of the door 2 is provided with a second end 20b which is shaped to be combined with the abutment surface 11 of the second part 2b, at least when the door 3 is in its closed position.

Furthermore, the abutment surface 11 comprises a hook element 12 jutting from the abutment surface 11 and adapted to limit the displacement of the door 3 along a direction orthogonal to the sliding one X, at least when said door 3 is in its closed position. In particular, this hook element 12 is arranged from the outer planar surface 2e of the frame 2 at a distance substantially equal to the thickness of the central body 2. This ensures that, following an increase of pressure inside the sterilization chamber 100, the door 3 remains exactly in its closing position thereby guaranteeing the sealing and the required mechanical strength.

As shown in the attached figures (see, in particular, figures 2, 9, 10a and 10b), the frame 2 further comprises a third part 2c arranged on a third edge 101c of the opening 101 and provided with a groove 60 operatively facing the outer side of the opening 101. The door 3 further comprises an additional portion 25 constrained by screws to a third end 20c of the central body 20 and provided with another hook element 26 operatively facing the inside of the opening 101 and couplable by abutment with the groove 60 of the third part 2c in order to limit the displacement of the door 3 along a direction orthogonal to the sliding one X, when said door is in its closed position.

As shown in figures, the linear guides 10 of the frame 2 are of the U-shaped type and the two linear profiles 22 of the door 3 are shaped so as to slide in this U-shaped profile. The workings to obtain the linear profiles 22 and the respective U-shaped linear guides 10 are simple and do not require particularly complex processes. Furthermore, the two first portions 21 and the additional portion 25 comprise a U-section having the same size and cross shape. In this way, as it will be more clear from the method of manufacturing the device 1, for making the two first portions 21 and the additional portion 25, it is just required starting from a section having proper length and then cutting it into three parts of predetermined length. In this way not only the production complexity of the door 3 is greatly reduced but also the waste of material used in manufacturing is reduced.

It should be also specified that, thanks to the particular U-shape, the two first portions 21 as well the additional portion 25 may be constrained to the respective ends 20a, 20c of the central body 20 without requiring any special cautions, but by using the second leg 40, 41 of the U-section as centering element respectively of the two first portions 21 and the additional portion 25, and then by mounting them by the concave part. It should be noted that the first leg of the U-section is either the first linear profile 22, in case of the two first portions 21, or the additional hook element 26 in case of the additional portion 25.

These two first portions 21 and this additional portion 25 are then constrained to the respective ends of the central body 20 by detachable connecting means such as, for example, screws.

It should be noted that, although an embodiment in which the door 3 slides with respect to the frame 2 in horizontal direction is depicted herein, however an embodiment in which the door 3 slides in vertical direction still falls within the protection scope of the present invention.

The method for making, or manufacturing, the above described opening/closing device 1, or anyway according to one or more of claims 1 to 9, will be described hereinafter.

This method comprises the steps of a) making the frame 2 of framing type and the step of b) making the door 3 slidingly combinable with the frame 2. According to the method, the step a) comprises the step of a1) making each of the two linear guides 10 operatively facing the outer side of the opening 101, at the two first parts 2a of the frame 2; and the step b) comprises:
- the step of b1) making a plate with substantially constant thickness to form the central body 20;
- the step of b2) making the two first portions 21 each provided with a first linear profile 22 operatively facing the inside of the opening 101 and sliding along the respective linear guide 10 of the two first parts 2a, at least when the door 2 is assembled to the frame 2; and
- the step of b3) constraining the two first portions 21 to the respective first end 20a of the central body 20.

This structural solution allows the device 1 for opening/closing autoclaves 100 to be built in a considerably simpler way. In fact, the door 3 is made by assembling elements not requiring any special working, together with the fact that even the same frame 2 of the device 1 requires extremely simple workings and, in any case, no working is carried out on the inner side of the frame 2, the one facing the opening 101, which would cause excessive complications to make both the frame 2 itself and, as a result, the door 3.

In a particular manner, it should be pointed out that said two first linear guides 10 are provided along the whole extent of the two first parts 2a, therefore through a considerably simplified working with respect to the past.

Still according to the method, before step a1), the step a) of the method further comprises the step of a0) making a substantially quadrangular and central hole 15 on a plate with substantially constant thickness.

Alternatively, before said step a1), the step a) of the method further comprises the step of a0') welding two first linear parts 2a with a second part 2b and a third part 2c, one to another, to form the frame 2 of framing type. This alternative method is not shown in the accompanying figures.

Again, for both the embodiments described above, after said step a1) it is comprised the step of a2) combining, for example by screws, an abutment surface 11 with the second part 2b of the frame 2, in order to limit the sliding of the door 2 in the closed position thereof.

Furthermore, said step a) comprises the step of a3) making a groove 60 operatively facing the outer side of the opening 101 at the third part 2c, and the step b) further comprises the step of b4) making an additional portion 25 provided with an additional hook element 26 operatively facing the inside of the opening 101 and couplable by abutment with the groove 60 of said at least one third part, in order to limit the displacement of the door 3 along a direction orthogonal to the sliding one X, when the door 3 is in its closed position, and the step of b5) constraining the additional portion 25 to a third end 20c of the central body 20, for example by screws or the like.

Furthermore, it should be also highlighted that the two first portions 21 and the additional portion 25 are made from a U-section having the same size and cross shape, conveniently cut to make those three portions 21 and 25. In this way, as above said, the manufacture of the door 3 is made much easier because both the working for making the plate-shaped central body 20 with constant thickness and the workings to obtain the linear profiles 22, are not particularly complex. Furthermore, the two first portions 21 and the additional portion 25 comprise a U-section having the same size and cross shape. In this way, thanks to the particular U- shape, the two first portions 21 as well as the additional portion 25 may be constrained to the respective ends 20a, 20c of the central body 20 without requiring any special cautions, but by using, as centering element, one of the two legs 40, 41 of the U-section (the second leg in fact is either the first linear profile 22, in case of the two first portions 21, or the additional hook element 26, in case of the additional portion 25), and then mounting them quickly by the concave part at the respective ends of the central body 20.

## Claims

1. Device (1) for opening/closing an autoclave (100) having an opening (101) for inputting/outputting one or more products to be sterilized, said device (1) comprising a frame (2) arranged at the edges (101a, 101b, 101c, 101d) of said opening (101), and at least one door (3) combinable with said frame (2) by sliding between a closed position and an open position, and vice versa, **characterized in that** said frame (2) is of framing type and comprises at least one first part (2a) arranged on a first edge (101a) of said opening (101) and provided with at least one linear guide (10) operatively facing the outer side of said opening, and **in that** said door (3) comprises a central body (20) and at least one first portion (21) provided with at least one first linear profile (22) operatively facing the inside of said opening and sliding along said linear guide (10) of said at least one first part, said at least one first portion (21) being distinct from said central body (20) and being constrained along at least one first end (20a) of said central body (20).

2. Device according to claim 1, **characterized in that** said frame (2) comprises at least one second part (2b) arranged at a second edge (101b) of said opening and provided with at least one abutment surface (11) to limit the sliding of said door (3) in the closed position thereof.

3. Device according to one or more of the preceding claims, **characterized in that** said at least one abutment surface (11) is constrained to said at least one second part (2b) of said frame (2).

4. Device according to claim 2 or 3, **characterized in that** said central body (20) is further provided with at least one second end (20b), said at least one second end (20b) being shaped to be combined with said abutment surface (11) of said at least one second part (2b), at least when said door is in its closed position.

5. Device according to one or more of the preceding claims, **characterized in that** said central body (20) comprises at least one plate with constant thickness.

6. Device according to one or more of the preceding claims, **characterized in that** said at least one abutment surface (11) comprises at least one hook element (12) jutting from said abutment surface (11) and adapted to limit the displacement of said door along a direction orthogonal to the sliding one (X), at least when said door (3) is in its closed position, said at least one hook element (12) being arranged from the outer planar surface (2e) of said frame (2) at a distance substantially equal to the thickness of said central body.

7. Device according to claim 1 to 6, **characterized in that** said frame (2) comprises a third part (2c) arranged on a third edge (101c) of said opening (101) and provided with a groove (60) operatively facing the outer side of said opening, and **in that** said door (3) comprises at least one additional portion (25) constrained to a third end (20c) of said central body (20) and provided with another hook element (26) operatively facing the inside of said opening and couplable by abutment with said groove (60) of said at least one third part (2c) in order to limit the displacement of said door along a direction orthogonal to the sliding one (X), when said door is in its closed position.

8. Device according to one or more of the preceding claims, **characterized in that** said at least one linear guide (10) of said frame is U-shaped and said at least one linear profile (22) of said at least one first portion (21) is shaped to slide in said U-shaped profile.

9. Device according to any one or more of the preceding claims, **characterized in that** said at least one first portion (21) and said at least one additional portion (25) comprise a U-section having the same size and cross shape.

10. Method for making an opening/closing device according to one or more of claims 1 to 9, comprising the step of a) making said frame (2) of framing type and the step of b) making said door (3) slidingly combinable with said frame (2), **characterized in that** said step a) comprises the step of a1) making at least one linear guide (10) operatively facing the outer side of said opening at said at least one first part of said frame (2a); and **in that** the step b) comprises the steps of: b1) making a plate with substantially constant thickness to form said central body (20); the step of b2) making at least one first portion (21) provided with at least one first linear profile (22) operatively facing the inside of said opening and sliding along said linear guide of said at least one first part, at least when the door is assembled; and the step of b3) constraining said at least one first portion on at least one first end (20a) of said central body (20).

11. Method according to claim 10, **characterized in that** before said step a1), the step a) further comprises the step of a0) making a substantially quadrangular and central hole on a plate with substantially constant thickness.

12. Method according to claim 10, **characterized in that** before said step a1), the step a) further comprises the step of a0') welding two first linear parts (2a) with a second part (2b) and a third part (2c), one to another, to form said frame (2) of framing type.

13. Method according to claim 10 and 11 or 12, **characterized in that** after said step a1) there is the step of a2) combining at least one abutment surface (11) with said second part (2b) of said frame (2), in order to limit the sliding of said door (2) in the closed position thereof.

14. Method according to one or more of claims 10 to 13, **characterized in that** said step of a) further comprises the step of a3) making at least one groove (60) operatively facing the outer side of said opening (101) at said at least one third part (2c), and **in that** said step b) further comprises the step of b4) making at least one additional portion (25) provided with at least one additional hook element (26) operatively facing the inside of said opening and couplable by abutment with said groove (60) of said at least one third part, in order to limit the displacement of said door along a direction orthogonal to the sliding one (X), when said door is in its closed position, and the step of b5) constraining said at least one additional portion (25) to a third end (20c) of said central body (20).

15. Method according to any one or more of claims 10 to 14, **characterized in that** said at least one first portion (21) and said at least one additional portion (25) are made from a conveniently cut U-section having the same size and cross shape.

16. Method according to one or more of claims 10 to 15, **characterized in that** said at least one first linear guide (10) is made along the whole extent of said at least one first part (2a).
